# EUROPEAN PATENT APPLICATION

(11) **EP 3 040 929 A1**
(43) Date of publication of application: **06.07.2016**
(21) Application number: 15200366.1
(22) Date of filing: 16.12.2015
(51) Int. Cl.: G06Q 30/06, H04L 29/08

(54) **ELECTRONICALLY ASSISTED CLOTHING SELECTION BASED ON PURPOSE AND LOCATION**

(30) Priority: 31.12.2014 US 201414587565
(71) Applicant: Nokia Technologies Oy, 02610 Espoo (FI)
(72) Inventor: LEPPÄNEN, Jussi, 33580 Tampere (FI); LEHTINIEMI, Arto, 33880 Lempäälä (FI); ERONEN, Antti, 33820 Tampere (FI)
(74) Representative: Nokia Corporation

(57) **Abstract**

A technique for clothing selection is provided. According to an example embodiment, the technique comprises obtaining activity information that identifies a type of an activity a user is planning to carry out; obtaining ambience information pertaining to a location of said activity, which ambience information characterizes ambient conditions in said location; deriving, on basis of said ambience information in dependence of said activity information, a target value for a clothing indicator that is descriptive of a required overall insulation level provided by one or more garments for said activity; receiving, over a wireless connection from one or more garments provided with wireless communication means, a respective value of said clothing indicator assigned for a garment selected by the user; deriving, for said activity, an accumulated value of the clothing indictors received from said garments selected by the user; and providing, via a user interface, on basis of a comparison of said accumulated value and said target value, an indication regarding a suitable clothing for said activity having been reached

## Description

### TECHNICAL FIELD

The example and non-limiting embodiments of the present invention relate to a method and to a system for assisting a user to select a suitable clothing in dependence of information that represents level of insulation provided by a piece of clothing.

### BACKGROUND

Choosing what to wear when going out may provide a challenge in terms of choosing a suitable amount and type of clothing. While this challenge is relevant to any activity that takes place outdoors, especially choosing a suitable amount and type of clothing to wear for manual work or for a sports activity taking place outdoors may be a particular challenge in terms of keeping one warm enough while on the other hand ensuring that the clothing is not too warm to cause discomfort or even dehydration. This challenge may especially pronounced in an unfamiliar geographical location where e.g. the humidity and/or short-term changes in the temperature may be different from those one is accustomed to.

### SUMMARY

According to an example embodiment, a method for clothing selection in an electronic device is provided. The method comprises obtaining activity information that identifies a type of an activity a user is planning to carry out; obtaining ambience information pertaining to a location of said activity, which ambience information characterizes ambient conditions in said location; deriving, on basis of said ambience information in dependence of said activity information, a target value for a clothing indicator that is descriptive of a required overall insulation level provided by one or more garments for said activity; receiving, over a wireless connection from one or more garments provided with wireless communication means, a respective value of said clothing indicator assigned for a garment selected by the user; deriving, for said activity, an accumulated value of the clothing indictors received from said garments selected by the user; and providing, via a user interface of said electronic device, on basis of a comparison of said accumulated value and said target value, an indication regarding a suitable clothing for said activity having been reached.

According to another example embodiment, an apparatus for clothing selection is provided. The apparatus comprises at least one processor and a memory storing a program of instructions, wherein the memory storing the program of instructions is configured to, with the at least one processor, cause the apparatus to at least: obtain activity information that identifies a type of an activity a user is planning to carry out; obtain ambience information pertaining to a location of said activity, which ambience information characterizes ambient conditions in said location; derive, on basis of said ambience information in dependence of said activity information, a target value for a clothing indicator that is descriptive of a required overall insulation level provided by one or more garments for said activity; receive, over a wireless connection from one or more garments provided with wireless communication means, a respective value of said clothing indicator assigned for a garment selected by the user; derive, for said activity, an accumulated value of the clothing indictors received from said garments selected by the user; and provide, via a user interface of the apparatus, on basis of a comparison of said accumulated value and said target value, an indication regarding a suitable clothing for said activity having been reached.

According to another example embodiment, a computer program is provided, the computer program comprising computer readable program code configured to cause performing, when said program code is run on a computing apparatus, a method comprising obtaining activity information that identifies a type of an activity a user is planning to carry out; obtaining ambience information pertaining to a location of said activity, which ambience information characterizes ambient conditions in said location; deriving, on basis of said ambience information in dependence of said activity information, a target value for a clothing indicator that is descriptive of a required overall insulation level provided by one or more garments for said activity; receiving, over a wireless connection from one or more garments provided with wireless communication means, a respective value of said clothing indicator assigned for a garment selected by the user; deriving, for said activity, an accumulated value of the clothing indictors received from said garments selected by the user; and providing, via a user interface of said electronic device, on basis of a comparison of said accumulated value and said target value, an indication regarding a suitable clothing for said activity having been reached.

The computer program referred to above may be embodied on a volatile or a nonvolatile computer-readable record medium, for example as a computer program product comprising at least one computer readable non-transitory medium having program code stored thereon, the program which when executed by an apparatus cause the apparatus at least to perform the operations described hereinbefore for the computer program according to an example embodiment of the invention.

The exemplifying embodiments of the invention presented in this patent application are not to be interpreted to pose limitations to the applicability of the appended claims. The verb "to comprise" and its derivatives are used in this patent application as an open limitation that does not exclude the existence of also unrecited features. The features described hereinafter are mutually freely combinable unless explicitly stated otherwise.

Some features of the invention are set forth in the appended claims. Aspects of the invention, however, both as to its construction and its method of operation, together with additional objects and advantages thereof, will be best understood from the following description of some example embodiments when read in connection with the accompanying drawings.

### BRIEF DESCRIPTION OF FIGURES

The embodiments of the invention are illustrated by way of example, and not by way of limitation, in the figures of the accompanying drawings, where
Figure 1 schematically illustrates some components of an arrangement according to an example embodiment,
Figure 2 illustrates a method according to an example embodiment, and
Figure 3 schematically illustrates some components of an apparatus according to an example embodiment.

### DESCRIPTION OF SOME EMBODIMENTS

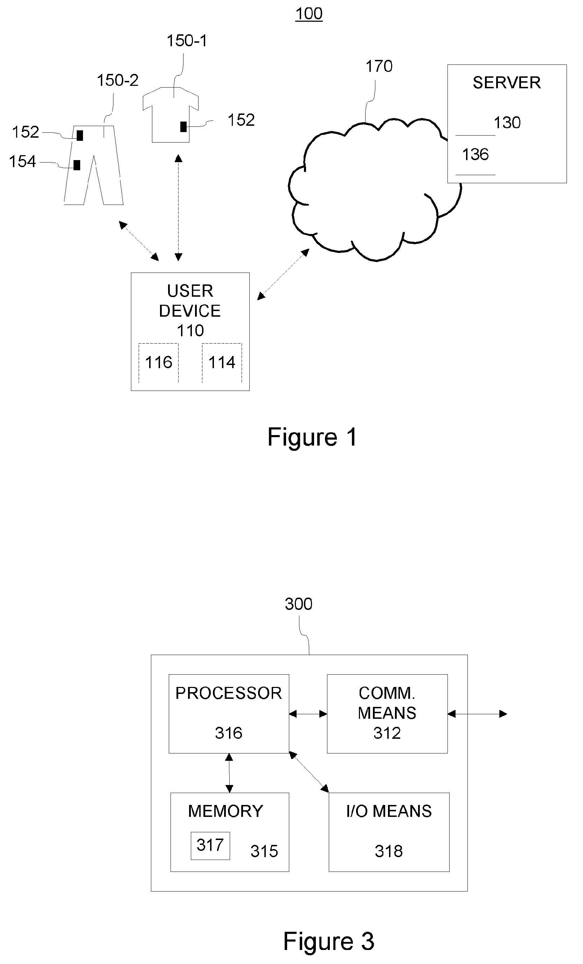

Figure 1 schematically illustrates some components of an exemplifying arrangement 100 that is suitable for providing a clothing selection arrangement according to one or more example embodiments of the present invention. In this regard, in the arrangement 100 a user device 110, a server 130 and garments 150-1 and 150-2 representing a plurality of garments 150 are depicted. In the framework of the arrangement 100, the clothing selection arrangement may be jointly provided by respective software means and hardware means provided in each of the user device 110, the server 130 and the plurality of garments 150, possibly together with further entities that are not depicted in Figure 1. Examples of such further entities include one or more further servers that may be employed to obtain complementary information, such as weather information. The components of the arrangement 100 are depicted with some sub-components or elements that will be described later in this text.

The user device 110 may comprise an electronic device such as a mobile phone, a portable navigation device, a media player device, a tablet computer, a laptop computer, etc. The user device 110 may comprise software means that is executable by hardware means of the user device 110. In particular, the software means provided in the user device 110 may comprise a client-side part of software means that is arranged to provide the clothing selection arrangement. This client-side part of the software means may be provided e.g. as one or more computer programs that are executable in the user device 110. These one or more computer programs may be referred to in the following as client software or as a client application.

The server 130 may comprise one or more devices (e.g. computers) one or more of which comprise software means executable by the respective device. In particular, the software means provided in the device(s) constituting the server 130 may comprise a server-side part of software means that is arranged to provide the clothing selection arrangement. This server-side part of the software means may be provided e.g. as one or more computer programs that are executable by the devices constituting the server 130. These one or more computer programs may be referred to in the following as server software or as a server application. The server application may be arranged to facilitate access to a remote clothing selection database 136, which may be stored in a memory and/or in a mass storage device that is provided in the server 130 or that is otherwise accessible by the server 130. The role and information content of the remote clothing selection database 136 is described in more detail later in this text.

The user device 110 and the server 130 may communicate with each other using wireless communication means, wired communication means or a combination thereof. In this regard, Figure 1 depicts an example where the server 130 is connected to a network 170, and the user device 110 is able to connect to the network 170 using wireless communication means (such as WLAN or cellular data) provided in the user device 110, thereby enabling communication with the server 130. Alternatively, the user device 110 may include wired communication means for connecting to the network 170 and hence enabling communication with the server 130.

In a typical scenario the server 130 is provided by a service provider in one or more server devices that are in control of a service provider that control access to the server 130 by the user device 130 and by other user devices. In another example scenario, the server 130 may be provided in a device that is under control of the user of the user device 130, typically. In such a scenario, the server 130 may be provided in another device that is under control of the user of the user device 130 or even in the user device 110 (e.g. the user device 110 may comprise also the server-side part of software means that is arranged to provide the clothing selection arrangement). Hence, in the latter example scenario the remote database 136 is not necessarily remote in a geographical sense but the term 'remote' indicates the fact that the database 136 may not be directly accessible by the client-side part of software means that is arranged to provide the clothing selection arrangement.

Each of the plurality of garments 150 comprises at least wireless communication means 152 and it may further comprise sensor means 154. A garment 150 or a piece of clothing that comprise at least the wireless communication means 152 may be referred to as a smart garment or as smartwear. In the example arrangement 100, the garment 150-1 is provided only with the wireless communication means 152, whereas the garment 150-2 is provided with both the wireless communication means 152 and the sensor means 154 (that are communicatively coupled to each other). The wireless communication means 152 may be provided as a wireless communication apparatus that enables short-range wireless point-to-point communication with the user device 110 using a suitable communication technology known in the art, such as near field communication (NFC) technology or Bluetooth Low Energy (BLE). The sensor means 154 may comprise one or more sensors for measuring characteristics that may be applied to detect a state that indicates that the user has put on the garment 150 and/or to detect environmental conditions. Examples of suitable sensors include a temperature sensor, a humidity sensor (e.g. a humistor), a proximity sensor, a barometer, an illumination sensor, etc.

Each of the garments 150 is further provided with a memory for storing information pertaining to the respective garment 150. The wireless communication means 152 is able to read from and write to the memory. The memory may be provided, for example, as part of the wireless communication means 152 or in a dedicated separate component that is communicatively coupled to the other components in the garment 150. In case the sensor means 154 is included in the garment 150, the sensor means 154 is at least able to write to and possibly also able to read from the memory, thereby enabling the sensor means 154 to store measurement data in the memory. In case the sensor means 154 is included, an additional option is to provide the memory as part of the sensor means 154.

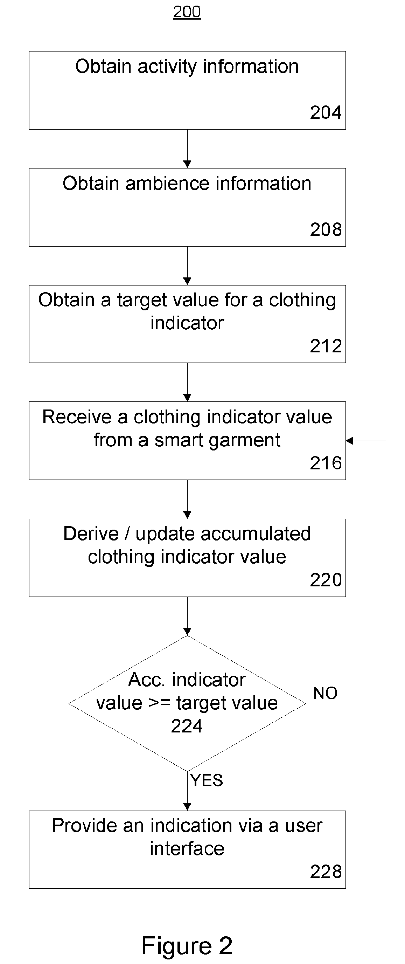

In the framework of the arrangement 100, the user device 110 may be arranged to implement a method 200 illustrated in Figure 2. As an example, the method 200 may be provided in the user device 110 as a computer program comprising computer readable program code (e.g. computer readable instructions) that causes, when executed, the user device 110 to carry out method steps described in the following. In other words, the method 200 may be implemented as part of the client software in the user device 110.

Operation of the method 200 may be initiated by a user action, e.g. via a user interface of user device 110. Once initiated, the method 200 proceeds to obtaining activity information, which may comprise one or more activity indications, as shown in block 204. The activity information serves as characterization of the type and/or physical effort associated with an activity the user is planning to carry out. As an example in this regard, the activity information may comprise an activity indication that identifies one of predefined activities, such as one of "standing/sitting", "walking", "jogging", "running", "cycling" etc. The predefined activity may be further mapped (e.g. by using a predefined mapping rule) to a numerical value that is representative of the associated physical effort in a predefined scale. As another example, the activity information may (additionally or alternatively) comprise an activity indication that identifies the level of physical effort associated with the user's planned activity as one of predefined effort levels, e.g. as one of very light", "light", "moderate", "hard", "extreme", which may be further mapped to a numerical value that is representative of the associated physical effort in a predefined scale. As a further example, the activity information may (alternatively or additionally) comprise an activity indication that directly indicates the level of physical effort associated with the user's planned activity as a numerical value in a predefined scale, e.g. as value in a range from 0 to 1, where 0 indicates a negligible (or a minimum) physical effort and 1 indicates an extreme (or a maximum) physical effort.

The activity information may be obtained e.g. from another application executed in the user device 110. Examples of such other applications include an electronic calendar or exercise tracker software where user-inputted entries may provide information that either directly provides the activity indication or that can be applied as basis for deriving the activity indication. As another example, the activity information may be obtained from an external information source that is available e.g. in a server accessible by the user device 110 via the network 170. Such an external information source may be provided e.g. as a database or an electronic calendar that defines one or more appointments or a daily schedule for schedule of a group or team the user belongs to. As a further example, the activity information may be received as direct use input via the user interface of the user device 110 in the course of execution of the method 200.

The method 200 may further comprise obtaining information characterizing ambient conditions concerning the location of interest, as indicated in block 204. For brevity and editorial clarity, in the following we refer to the information that characterizes the ambient conditions as ambience information. As an example, the ambience information may comprise weather information, as indicated in block 208. The obtained weather information pertains to a location of interest at a time of interest. The location of interest indicates the location of the planned activity. Typically, this is the user's current geographical location, which may be obtained using positioning means (e.g. a GPS receiver) that may be included in the user device 110 or that may be otherwise available for the user device 110. Alternatively, the location of interest may be another geographical location defined by the user (e.g. via the user interface of the user device 110), e.g. a location different from the user's current location where the planned activity is to take place, a destination the user is planning to visit in the near future such as a destination of a business trip or a holiday trip.

Obtaining the weather information may comprise the user device 110 accessing a weather service available in a server that may be available via the network 170. In this regard, the user device 110 may request and receive the weather information pertaining to the location of interest from the server. Obtaining the (requested and) received weather information may comprise information that is indicative of current, possibly observed or measured, weather conditions. Alternatively or additionally, the (requested and) received weather information may comprise information that is indicative of predicted, current or future, weather conditions at a moment of interest. A request for the weather information sent from the user device 110 may comprise an indication of the moment of interest. The available and/or received weather information may include one or more pieces of information concerning measured and/or predicted values for one or more of the following weather parameters: temperature, absolute humidity, relative humidity, precipitation status (e.g. drizzle, rain, sleet, snow, graupel, hail or no precipitation), wind velocity, etc. A request for the weather information sent from the user device 110 may comprise indication(s) of the weather parameter(s) of interest.

As another example, the user device may comprise a sensor means 114 that comprises one or more sensors for measuring environmental characteristics that may applied to derive one or more weather parameters. Examples of suitable sensors include a temperature sensor, a humidity sensor (e.g. a humistor), a proximity sensor, a barometer, an illumination sensor, etc. Hence, obtaining the weather information may comprise the user device 110 receiving one or more pieces of weather information (e.g. a measured temperature and/or a measured humidity) from the sensor means 114.

The weather information typically provides relevant pieces of information for an activity that takes place outdoors. For an activity that takes place indoors, e.g. in a sports hall, in an ice hall, in a gym, etc. the ambience information may be descriptive of the indoor conditions such as temperature and/or absolute or relative humidity. Moreover, for an activity that takes place indoors the information that indicates the location of interest may comprise, in addition to or instead of an indication of a geographical location, an indication of the type of the (indoor) location. The type of the indoor location may be indicated the location type to be one of predefined location types. As an example, the one or more predefined location types may include one or more of the following: "a sports hall", "an ice hall, "a swimming hall", "a gym", etc.

Herein, the term clothing indicator refers to a metric that is descriptive of a level of thermal insulation or insulation of other type provided by a piece of clothing. Hence, a clothing indicator value assigned to an individual garment 150 is descriptive of the level of (thermal or other) insulation the garment 150 is estimated to provide for the user wearing the garment 150.

An example of a clothing indicator suitable for the purpose is the clo-value, defined e.g. in Gagge AP, Burton AC, Bazett HC: A practical system of units for the description of the heat exchange of man with his environment. Science 1941, 94(2445):428-430, which is incorporated herein in its entirety. The clo-value for a garment is a metric that is descriptive of the amount of thermal insulation the garment is estimated to provide for the user wearing the garment. In other words, the clo-value is descriptive of the amount of thermal insulation a garment is able to provide between the user and his/her environment. Another example of the clothing indicator suitable for the purpose is a windproofness value, which may be employed to indicate the extent of windproofness of a garment as a numerical value in a predefined scale (e.g. from 0 to 1) or simply as an indication regarding whether a garment is windproof or not (e.g. as 0 or 1). A further example of the clothing indicator suitable for the purpose is a waterproofness value, which may be employed to indicate the extent of waterproofness of a garment as a numerical value in a predefined scale (e.g. from 0 to 1) or simply as an indication regarding whether a garment is waterproof or not (e.g. as 0 or 1).

An overall value for a clothing indicator that is descriptive of the overall level of (thermal or other) insulation provided for the user may be derived as a combination of the respective clothing indicator values assigned for the individual garments 150 the user is wearing. As an example in this regard, the overall level of insulation may be derived on basis of the sum of the clo-values assigned for the individual garments 150 the user is wearing, e.g. by employing the sum as such or by multiplying the sum by a predefined factor. As an example in this regard, the article by E.A. McCullough, B.W. Jones., J. Huck, titled "A comprehensive Data Base for Estimating Clothing Insulation" (downloadable at the priority date of this patent application e.g. at http://www.cbe.berkeley.edu/research/other-papers/McCullough%20et%20al%201985%20A%20comprehensive%20data%20bas e%20for%20estimating%20clothing%20insulation.pdf), incorporated herein by reference in its entirety, refers on page 35 to various estimates for the overall insulation. According to this referred disclosure, the overall level of (thermal) insulation might be estimated by multiplying the sum of the individual clothing indicator values by the factor 0.82. Therefore, a suitable (or sufficient) overall level of clothing for the user may be defined as a target value for a given clothing indicator, which target value represents a required (or a minimum) overall value for the given clothing indicator. In the framework of the method 200, the target values may hence comprise, for example, one or more of the following: a target value for the clo-value, a target value for the windproofness value and a target value for the waterproofness value.

In this regard, the method 200 further comprises obtaining target values for one or more clothing indicators that indicate respective overall levels of thermal insulation or insulation of other type considered suitable under the circumstances, as indicated in block 212. Obtaining the target value(s) may comprise deriving a local target value for one or more clothing indicators on basis of the clothing selection information available in the user device 110 and using the local target value(s) as the respective target value(s). As another example, obtaining the target value(s) may comprise obtaining a reference value for one or more clothing indicator(s), where the reference values(s) are derived on basis of the clothing selection information available in the remote clothing selection database 136 and using the reference value(s) as the respective target value(s). As a further example, obtaining the target value(s) may comprise deriving the local target value(s) and the respective reference value(s) and deriving the target value(s) on basis of the local target value and the respective reference value. Each of the local target values and the reference values is derived at least in part on basis of the ambience information, e.g. the weather information. Moreover, In case the activity information is obtained and is hence available, at least some of the local target values and the respective reference values may be derived in dependence of the activity information.

Deriving a target value on basis of the local target value and the respective reference value may comprise deriving the target value as an average or as a weighted average of the respective local target value and the corresponding reference value. As another example, deriving a target value on basis of the local target value and the respective reference value may comprise selecting a predefined one of the local target value and the respective reference value in case the deviation (e.g. a difference) between the two exceeds a predefined threshold.

The respective target values for one or more clothing indicators may be (jointly) referred to as clothing information. The clothing information may further comprise recommend (or required) combination of garment types, as will be described later in this text.

Deriving the local target value for one or more clothing indicators may comprise deriving the one or more target values on basis of clothing selection information provided in the user device 110. This locally-available clothing selection information may be provided as a local clothing selection database 116 stored in a memory and/or a mass storage device in the user device 110. The local clothing selection database 116 may include a mapping table or an information structure of other type that is suitable for defining a mapping between ambience information and a respective target value for one or more clothing indicators. The target value(s) for the clothing indicator(s) are hence descriptive of the overall level of clothing considered suitable (or sufficient) for the upcoming activity in view of the information stored in the local clothing selection database 116.

The information stored in the local clothing selection database 116 may comprise generic clothing selection information that may be derived on basis of experimental data collected from a (high) number of users carrying out activities of various type in various ambient conditions and in various locations. Such generic clothing selection information is applicable to any given user. The information stored in the local clothing selection database 116 may, additionally or alternatively, comprise clothing selection information that is at least in part specific to the user of the user device 110, derived e.g. on basis of previous activities of the user.

According to a straightforward example, the local clothing selection database 116 comprises clothing selection information that defines a mapping between a temperature and a corresponding target value for the clo-value. Hence, herein the temperature serves as an example of the ambience information and the clo-value serves as an example of the clothing indicator Table 1 provides a non-limiting example for such mapping information. According to another example, the local clothing selection database 116 comprises clothing selection information that defines a mapping between a temperature and a corresponding target value for the clo-value in dependence of the activity indication. Table 2 provides a non-limiting example for such mapping information.

**Table 1**

| **Temperature** | **Clo-value** |
|---|---|
| 21 C | 0.7 |
| 10 C | 1.5 |
| 1 C | 2.3 |
| -18 C | 3.5 |

**Table 2**

| **Temperature** | **"standing"** | **"slow walking"** | **"fast walking"** |
|---|---|---|---|
| 21 C | 1.5 | 0.7 | 0.3 |
| 10 C | 3.1 | 1.5 | 0.7 |
| 1 C | 4.7 | 2.3 | 1.1 |
| -18 C | 7.1 | 3.5 | 1.7 |

In further examples of the clothing selection information included in the local clothing selection database 116 one or more additional pieces of ambience information, e.g. weather parameters, are taken into account in the mapping between the ambience information and the corresponding target value for the clothing indicator, e.g. the clo-value, either independently of (e.g. without considering) the activity indication or in dependence of the activity information.

Along similar lines, the local clothing selection database 116 may include e.g. clothing selection information that provides a mapping between wind velocity and a corresponding target value for the windproofness value, where the mapping is provided either independently or in dependence of the activity information. This mapping may further take into account one or more other weather parameters, e.g. the temperature. Furthermore, the local clothing selection database 116 may include clothing selection information that provides a mapping between precipitation status and a corresponding target value for the waterproofness value, where the mapping is provided either independently or in dependence of the activity information. This mapping may further take into account one or more other weather parameters, e.g. the temperature, the absolute humidity and/or the relative humidity.

In further examples, the local clothing selection database 116 may further consider garment types required to reach suitable (or sufficient) level of clothing. As an example in this regard, the mapping may not only yield the target value(s) for the clothing indicator(s) but also provides a recommended combination of garment types in order to more accurately characterize the suitable (or sufficient) clothing. This information may be provided as one or more garment type indictors, each specifying the type of respective recommended garment. The garment type indicator(s) specifying the recommended combination of garment types may be provided as part of the clothing information.

A garment type indicator may indicate the garment 150 to belong to one of predefined garment categories. As an example, the predefined garment categories may include one or more of the following: "training shorts", "training top", t-shirt", "trousers, "socks", "gloves", "hat", "bra", "panties", "half-slip", "long underwear", "long underwear top", "pantyhose/stockings", "sandals", "shoes", "slippers", "boots", "blouse", "short sleeve dress shirt", "long sleeve dress shirt", "long sleeve flannel shirt", "sweatpants", "overalls", "coveralls", "thin skirt", "thick skirt", "thin sleeveless vest", "thick sleeveless vest", "sleeveless short gown", "pajamas", etc.

Obtaining the reference value for one or more clothing indicators may comprise (requesting and) receiving respective one or more reference values from the server 130. In this regard, the user device 110 may request and receive a reference value for one or more clothing indicators from the server 130, which server 130 is arranged to access the clothing selection database 136 to obtain the requested reference value(s). In this regard, the remote clothing selection database 136 may be arranged to store reference values for the clothing indicator(s) or information that enables deriving requested reference value(s) for the clothing indicator(s). As in case of the local database 116 described in the foregoing, the server 130 may also obtain (and provide to the user device 110) one or more garment type indicators that indicate a recommended combination of garment types together with the respective reference values for one or more clothing indicators. In this regard, the garment type(s) may be indicated as described in the foregoing in context of the local database 116.

The information stored in the remote clothing selection database 136 may comprise database entries that comprise one or more of the following data items:
- identity of a user having performed an activity associated with the database entry
- personal characteristics of the user (e.g. indications of weight, height and/or fitness level of the user)
- time and date of the activity associated with the database entry,
- location information, e.g. geographical location and/or the type of location, of the activity associated with the database entry,
- ambience information, e.g. weather information, pertaining to the activity associated with the database entry,
- activity information characterizing the activity associated with the database entry, defined e.g. as one or more activity indications,
- overall value of one or more clothing indicators that characterize the clothing worn by the respective user during the activity associated with the database entry, and
- garment type indicators and respective clothing indicators of the garments worn by the respective user during the activity associated with the database entry.

Consequently, when the server 130 receives a request to provide one or more reference values for the user device 110, the server 130 searches the remote database 136 in order to identify database entries that match the request, derives the requested reference value(s) on basis of the identified database entries and transmits the derived reference value(s) via the network 170 the user device 110. Derivation of the requested reference value(s) may comprise deriving an average or median of the overall values of the one or more clothing indicators provided in the database entries identified in the search. The server 130 may further derive the recommended one or more garment type indicators on basis of the identified database entries that represent the recommended combination of garment types. This may involve e.g. identifying a combination of garment types most commonly appearing among the identified database entries and using this combination of garment types as the recommended combination of garment types.

The user device 110 may include one or more personal characteristics of the user (e.g. indications of weight, height and/or fitness level of the user) in the request for the reference value(s) for the reference values(s) of clothing indicators transmitted to the server 130. In response to such a request, the server 130 searches the database 136 to identify database entries that indicate personal characteristics that are similar or substantially similar to those received in the request and derives the reference value(s) (and possibly also the recommended combination of garment types) on basis of the identified database entries. As another example, the request may further include one or more further characteristics pertaining to the activity the user is planning to carry out, e.g. one or more of the weather information, an indication of the time of the day, indication of the geographical location and/or the type of the location and the activity information. Consequently, in response to the request the server 130 searches the database 136 to identify database entries that indicate personal characteristics similar to those received in the request and that further indicate further characteristics that match those received in the request, and derives the reference value(s) on basis of the identified database entries. The reference value(s) for the clothing indicator(s) are hence descriptive of the overall level of clothing considered sufficient for the upcoming activity in view of the information pertaining to a number of users stored in the remote clothing selection database 136.

Once the target value(s) for the clothing indicators are obtained, the method 200 proceeds to deriving one or more accumulated clothing indicator values that are indicative of the combined clothing level provided by the garments 150 selected by the user. An accumulated clothing indicator value may be derived as a sum or as a product of the respective clothing indicator values assigned for one or more garments selected (e.g. worn) by the user, as described in the following.

Derivation of an accumulated clothing indicator value may comprise receiving, from each garment 150 selected (e.g. worn or to be worn) by the user, the value of the respective clothing indicator assigned for the garment 150, as indicated in block 216. As an example in this regard, each garment 150 may store the assigned value for one or more clothing indicator(s) in the memory provided in the garment 150 and transmit the relevant clothing indicator value(s) to the user device 110 using the wireless communication means 152. The garment 150 may further transmit additional information, e.g. a garment identity indicator that identifies the garment 150 and/or a garment type indicator. The garment identity indicator may uniquely identify the garment 150, whereas the garment type indicator may indicate the garment 150 to belong to one of predefined garment categories, e.g. along the lines described in the foregoing.

An event that indicates user-selection and hence triggers a garment 150 to transmit the clothing indicator value(s) (possibly together with further information, such as the garment identity indicator and/or the garment type indicator) may comprise the wireless communication means 152 (provided e.g. as a NFC target) detecting presence of the user device 110 (including e.g. a wireless communication means provided as a NFC initiator) due to the user bringing the user device 110 to a proximity (e.g. in a range from 1 cm to 20 cm) of the wireless communication means 152 in the garment 150 or vice versa. As another example, the event that indicates user-selection and hence triggers the garment 150 to transmit the assigned clothing indicator value(s) (possibly together with further information, such as the garment identity indicator and/or the garment type indicator) may comprise the sensor means 154 included in the garment 150 detecting a state that indicates that the user has put on the garment 150. For detection of such state the sensor means 154 may comprise, for example, one or more of a temperature sensor, a pressure sensor, a humidity sensor, a proximity sensor, a barometer and an illumination sensor, and the detection of the state that indicates the user having put on the garment 150 may comprise one or more of these sensors indicating a value in a first respective predefined range and/or indicating a change in value that falls within a second respective predefined range.

In response to receiving one or more clothing indicator values from one of the garments 150, the user device 110 computes or updates the accumulated clothing indicator value(s) by increasing the current accumulated value(s) by the newly received one(s), as indicated in block 220. In other words, the accumulated clothing indicator value(s) are increased each time new value(s) are received from a further garment selected by the user, e.g. each time transmission of the clothing indicator value(s) from one of the garments 150 is triggered. At the same time, if the garment identity indicator and/or the garment type indicator is received together with the clothing indicator value(s) from the garment 150, the user device 110 may also keep track of the identities and/or types of the garments 150 selected by the user.

The user device 110 provides an indication regarding suitable clothing having been reached on basis of a comparison between the accumulated clothing indicator value and a respective target value. As an example, when the accumulated clothing indicator value reaches or exceeds the respective target value (block 224), the user device 110 may provide an indication regarding the required (or sufficient) amount of clothing having been reached or exceeded, as indicated in block 228. This indication may be provided via a user interface of the user device 110. The indication may include for example audible indication, such as playing back an alarm sound via an audio reproduction means in the user device 110, displaying a visual indication in a display of the user device 110 and/or providing haptic indication e.g. by activating a vibratory component in the user device 110 for a short period of time. Additionally or alternatively, one or more of the garments 150 may comprise a vibratory component and the accumulated clothing indicator value reaching or exceeding the respective target value may result in the user device 110 instructing (e.g. by sending a message or command via the wireless communication means 152) the garment 150 to provide a haptic indication by activating the vibratory component for a short period of time as an indication regarding the required (or sufficient) clothing level having been reached or exceeded.

As another example, the user device 110 may provide a specific (e.g. different) audible, visual and/or haptic indication in response to the accumulated clothing indicator value being equal or substantially equal to (or within a predefined margin centered at) the respective target value. This approach enables distinguishing a state where just the required amount (e.g. optimal amount) of clothing having been reached from a state where excessive amount of clothing has been selected.

Alternatively or additionally, the user device 110 may provide an indication regarding the required (or sufficient) amount of clothing having not been reached when the accumulated clothing indictor value fails to reach the respective target value (block 244). In particular, such an indication may be provided e.g. upon computing or updating the accumulated clothing indicator value(s). As in case of the indication of block 228, also the indication regarding required amount of clothing having not been reached may be provided as an audible, visual and/or haptic indication via the user interface of the user device 110 or as a haptic indication by a vibratory component in one of the user-selected garments 150. If both the indication regarding the required (or sufficient) amount of clothing having been reached or exceeded and regarding the required (or sufficient) amount of clothing having not been reached are provided different audible, visual and/or haptic indications to ensure differentiating the two states.

In addition to the garments 150 representing the smartwear, the user may also select one or more garments that are not able to transmit a clothing indicator value for the user device 110. Herein, such a garment is referred to as a non-smart garment. In case of a non-smart garment, respective values for one or more clothing indicators are not directly available for incrementing the respective accumulated clothing indicator values. In case that the user selects a non-smart garment, the user may use the user device 110 to access a server (e.g. the server 130 or another server) via the network 170 in an attempt to obtain a respective value for one or more clothing indicators associated with the selected non-smart garment:
This may involve the user providing identification information for the non-smart garment under consideration to the user device 110, e.g. by inputting information available in a product label of the non-smart garment via the user interface of the user device 110 or by capturing an image depicting the product label. This identification information may e.g. provide an indication of the material of the non-smart garment under consideration. Additionally or alternatively, the user may input, via the user interface of the user device 110, a type of the non-smart garment which may be applied to specify a garment type indicator. The garment type indicator may be included in the identification information, and the garment type indicator may indicate the non-smart garment under consideration to belong to one of predefined garment categories, which predefined garment categories may include one or more of the exemplifying garment categories listed in the example provided in the foregoing.

The user device 110 may transmit, via the network 170 to said server (e.g. the server 130), a query or request concerning a value for one or more clothing indicator that may be assigned to the garment, which query or request includes the identification information for the non-smart garment under consideration provided by the user. The server attempts to obtain the requested the clothing indicator value(s) and transmits the requested value(s), if available, to the user device 110 via the network. Consequently, the user device 110 increments the accumulated clothing value(s) by the respective received clothing indicator values assigned to the non-smart garment under consideration.

The server attempting to obtain the requested clothing indicator value(s) may comprise the server access a database in the server in order find clothing indicator value(s) assigned for the garment identified in the query or request received from the user device 110. Alternatively, the server may consult another server or service to obtain the requested clothing indicator value(s). Identification of the clothing indicator value(s) to be provided to the user device 110 may be based on mapping between material(s) of the non-smart garment and/or type of the non-smart garment. Consequently, the database employed for this purpose may store a plurality of database entries that each provide a mapping between a material or a predefined combination materials and corresponding clothing value(s), a mapping between a type of garment and corresponding clothing value(s), or a mapping between a type of garment constructed of a predefined material or a predefined combination of materials and corresponding clothing value(s). Non-limiting examples of such mappings are found e.g. in the article by E.A. McCullough, B.W. Jones., J. Huck referred to in the foregoing.

During the activity, the user device 110 may optionally keep track of one or more characteristics that are indicative of the (thermal or other) insulation, windproofness and/or waterproofness of the clothing worn by the user. Examples of such characteristics include temperature and humidity under the clothing worn by the user. In this regard, the sensor means 114 that may be provided in the user device 110 may comprise e.g. a temperature sensor arranged to track the ambient temperature and/or a humistor arranged to track humidity, which sensor means 114 may be employed to track the temperature and/or humidity under the user's clothing in case the user carries the user device 110 with him/her during the activity. As another example, the sensor means 154 in one or more of the garments 150 worn by the user may include the temperature sensor and/or the humistor, and the temperature and/or humidity tracked by these sensors can be subsequently transferred to the user device 110 (e.g. by using the wireless communication means 152).

Consequently, once the user's activity is completed, the user device 110 may analyze the characteristics (e.g. the temperature and/or the humidity) tracked during the activity in order estimate the observed suitability (or sufficiency) of the level of clothing worn by the user. As an example in this regard, a value of a given characteristics falling within a respective predefined range may be considered as an indication of a suitability of the level of clothing and hence as an indication of correctly derived target value(s) for the applied clothing indicator(s), whereas the value of the given characteristic falling outside the respective predefined range may be considered as an indication of unsuitability of the level of clothing and hence as an indication of an incorrectly derived target value(s) for the applied clothing indicator(s). As a concrete example in this regard, a tracked temperature that is below a predefined temperature range may serve as an indication of insufficient level of clothing, whereas a tracked temperature that is above the predefined temperature range may serve as an indication of an excessive level of clothing.

After completion of the activity, the user device 110 may optionally provide information concerning the completed activity to the server 130 for storage in the remote clothing selection database 132. This information may be subsequently used in deriving the reference value(s) for the clothing indicator(s) for the user himself/herself and/or for other users making use of the clothing selection arrangement. Moreover, at the same time the user device may also update or complement the information stored in local clothing selection database 116 on basis of the information concerning the completed activity.

Provision of the information for storage in the remote clothing selection database 136 may comprise the user device 110 sending, to the server 130, a message that carries the information to be included in the database 136. The information carried in the message comprises at least the personal characteristics of the user (e.g. indications of weight, height and/or fitness level of the user), the ambience information pertaining to the activity (e.g. one or more or the temperature, the relative or absolute humidity, the wind velocity, the precipitation status) and the accumulated value(s) for the clothing indicator(s) derived in the user device 130 for the completed activity. The request may further comprise one or more of the following: identity of the user of the user device 110, time and date of the completed activity, geographical location of the completed activity, the location type pertaining to the location of the completed activity, the local target value(s) for the clothing indicator(s) derived in the user device 110 for the completed activity, activity information characterizing the completed activity, respective clothing indicator values of the garments worn by the user during the completed activity, and respective garment type indicators of the garments worn by the user during the completed activity.

Provision of the information for storage in the remote database 136 may be conditional to the result of the analysis carried after the user's activity: the information may be provided for storage in the remote database 136 only in case the analysis indicates suitability of the applied level of clothing. As another example in this regard, the information transmitted to the server 130 for storage in the remote database 136 may be complemented with the outcome of the analysis, e.g. whether the applied level of clothing (indicated by the accumulated value(s) for the clothing indicator(s)) was considered suitable or not (e.g. insufficient, suitable or too much). The server 130 may then store this piece of information together with the other information in the respective database entry of the remote database 136. Alternatively, the server 130 may adjust the other information received from the user device 110 in view of the indication of the outcome of the analysis before storing the information in the database 136, e.g. by scaling the indicated accumulated value(s) of the clothing indicator(s) towards a smaller value in case the level of clothing was indicated excessive and/or by scaling the indicated accumulated value(s) of the clothing indicator(s) towards a larger value in case the level of clothing was indicated insufficient.

After completion of the activity, the user device may optionally evaluate the accuracy of the clothing indicator value(s) assigned for a garment 150 worn during the activity. In this regard, the tracked characteristics that are indicative of the (thermal or other) insulation, windproofness and/or waterproofness of the clothing worn by the user during the activity may be employed. As described in the foregoing, such characteristics may be tracked e.g. by using the sensor means 114 in the user device 110 and/or sensor means 154 in one or more of the garments 150 worn during the activity.

As an example in this regard, after completion of an activity the temperature and/or humidity measured under the user's clothing during the activity may be stored in a database together with the weather information and the activity information pertaining to the activity. This database may be a local database in the user device 110 or a remote database e.g. in the server 130, where each database entry includes an indication of the date of activity, weather information associated with the activity, activity information characterizing the activity, identity indicators and respective clothing indicator value(s) for one or more garments 150 worn during the activity and the measured characteristics (e.g. temperature and/or humidity) indicative of the insulation provided by the worn garments 150.

The evaluation of the accuracy of the clothing indicator value(s) assigned for a garment 150 may involve searching the database to identify activities of similar or substantially similar kind (e.g. the same or similar activity, the same or similar weather) during which a certain garment 150 has been worn. In case the identified activities indicate a certain trend or a sudden change in one of the measured characteristics (e.g. a measured temperature that gradually decreases over time or a drop in measured temperature that exceeds a predefined threshold, respectively), the user device 130 device may conclude that the garment has worn and the clothing indicator value(s) assigned for the certain garment 150 indicates higher level of insulation that the certain garment 150 currently provides. Consequently, the user device 110 may compute and updated (decreased) clothing indicator value(s) and provide the updated value(s) to the certain garment 150, via the wireless communication means 152, for storage in the memory therein and for subsequent use as the clothing indicator value(s) assigned for the certain garment 150.

While the operation described in the foregoing in the framework of the arrangement 100 and/or in context of the method 200, may be assumed to imply a single, relatively short-term activity (of a few minutes to a few hours), it directly generalizes to an activity of an extended duration (of e.g. a few days).

However, in scenario where the selection of the garments 150 (blocks 216 and 220) is carried out for a time period of extended duration, this time period may involve a plurality of separate activities. Such a situation may occur e.g. when packing for a holiday or for a business trip. For such a case, the method 200 described in the foregoing may be varied such that the target value(s) of the clothing indicator(s) are derived such that they represent the combined overall value(s) of the respective clothing indicator(s) over all activities expected to fall within the time period of interest. Consequently, the user device 110 provides the indication regarding the required (or sufficient) amount of clothing having been reached or exceeded (block 220) in response to the garments 150 that are sufficient in view of all activities expected to occur during the time period of interest have been selected. The activity information that enables determining the number and/or timing of activities can be obtained as described above.

As described in the foregoing, at least some of the garments 150 may store in the memory the garment type indicator assigned thereto, and a garment 150 may be arranged to transmit the garment type indicator together with the clothing indicator value(s) when selected by the user, where the garment type indicator may indicate the garment 150 to belong to one of predetermined garment categories, which predetermined garment categories may include e.g. one or more of the exemplifying garment categories listed in the example provided in the foregoing. Moreover, the user device 110 may receive, from the local database 116 or from the server 130, the recommended combination of garment types for a given activity. Consequently, in another variation of the method 200, the evaluation in block 224 may be complemented with a further condition requiring that a recommended combination of garment types that results in accumulated value(s) of the clothing indicator(s) that reach or exceed the respective target value(s) is available for the planned activity or for a plurality of planned activities before providing the indication regarding the required (or sufficient) amount of clothing having been reached or exceeded via the user interface (block 228).

Such further evaluation (in block 224) may involve the user device 110 automatically arranging the user-selected garments 150 into combinations that are considered suitable for one of the planned activities in view of the respective recommended combination of garment types and proceeds into providing the indication of block 228 only in response to respective recommended combinations of garment types with sufficient clothing indicator values having been encountered. Consequently, a suggested combination of selected garments 150 will be provided for each of the planned activities. Such automatic arranging may be done, for example, as follows: the user device 110 obtains the conditions (e.g. the type of planned activity and/or the ambience information) at which clothing combinations are needed. It then proceeds to construct combinations of selected garments 150 for these conditions. Depending on the obtained conditions, there is a certain respective target value of one or more clothing indicators, e.g., a clo-value target, for each clothing combination. Moreover, the activity information may be applied to determine the type of clothing needed, such as clothing suitable for outdoor activities such as running or clothing suitable for indoor activities such as tennis or badminton. The user device 110 may obtain for each condition the selected garments 150 that match the respective activity (such as jogging outdoors), first starting, for example, from the outer garment layer (such as college pants or a college shirt). It may then proceed the underwear layer and the shoes. It may then compare the estimated clo-value to the target clo-value and either reduce or add garments to the activity depending on requirement indicated by their respective clo-value target. Moreover, for example if windproofness is required, the user device 110 will try to find a windproof outer layer garment (e.g. a selected garment 150 assigned a windproofness value that reaches or exceeds a predefined threshold) to be included the respective combination of garments. If a larger clo-value is required, the user device 110 may add more garments in between the outer layer and the underwear layer, until the target clo-value is reached or exceeded. If a lower clo-value is required, the system will try to use garments of type which are indicated as suitable for the same conditions but which have a smaller clo-value assigned therefor. For example, it may change a long sleeved garment to short sleeved one that has a smaller clo-value. The above procedure is repeated for all planned activities. In this variation the provision of the indication via the user interface (block 228) may further comprise indications of one or more suggested clothing combinations that identify the selected garments, possibly together with days/activities to which the user device 110 is suggesting them to be used. Such depiction could be done, for example, as listings of selected garments 150 or with graphical icons that are representative of respective selected garments 150. Moreover, the user device 110 may further provide, e.g. via display provided as part of the user interface of the user device 110, visual indications of the automatically arranged (e.g. suggested) combinations of selected garments 150 for some or all of the planned activities.

In a further variation of the previous variation of the method 200, i.e. making use of the garment type indicators, a basic frequency of changes of clothing may be considered instead of or in addition to the consideration of the planned activities. An indication regarding the basic frequency of change may be received e.g. via the user interface of the user device 110. The basic frequency of change may be e.g. one day. The basic frequency of change may be specified for the whole attire in combination or it may be specified separately for a number of garment types of interest. As a yet further variation to this approach, a re-use factor may be defined for some of the garments 150. The re-use factor may be employed to define the number of times a given garment may be worn (e.g. during a trip). The re-use factor for a given garment 150 may be received e.g. via the user interface of the user device 110. As another example, an automated or semi-automated re-use factor may be applied such that there is a predefined, possibly garment type specific, default value of the re-use factor (e.g. 1, implying that the garment 150 may be worn twice, i.e. one round of re-use after the initial use), which may be automatically downscaled to zero for one garment 150 of the respective type in response to observing a planned activity that involves (expected) physical effort that exceed a predefined threshold (and that is hence likely to result in excessive perspiration of the user). A semi-automated usage of the re-use factor may be provided e.g. by providing two or more preselected approaches: a first approach may apply a high default value for the re-use factor (e.g. 2 or 3) and a second approach may apply a low default value for the re-use factor (e.g. 0 or 1) - and the user may select either the first or the second approach via the user interface of the user device 110.

While the description above refers to clothing selection arrangement to be employed for selecting the garments 150 for the user of the user device 110, the described approach is equally applicable for selecting the garments 150 for another person, e.g. for a family member of a friend, or jointly for a number of persons.

Figure 3 schematically illustrates an exemplifying apparatus 300 upon which an embodiment of the invention may be implemented. The apparatus 300 as illustrated in Figure 3 provides a diagram of exemplary components of an apparatus, which is capable of operating as or providing the user device 110 according to an example embodiment. The apparatus 300 comprises a processor 316, a memory 315 and a communication means 312 including one or more communication apparatuses, such as a network card or a network adapter enabling wireless or wireline communication with another apparatus and/or radio transceiver enabling wireless communication with another apparatus over radio frequencies. The apparatus 300 may further comprise a user interface means 318 for providing data, commands and/or other input to the processor 310 and/or for receiving data or other output from the processor 310, the user interface 318 comprising for example one or more of a display, a keyboard or keys, a mouse or a respective pointing device, a touchscreen, etc. The apparatus 300 may comprise further components not illustrated in the example of Figure 3. As an example in this regard, the apparatus 300 may comprise the sensor means 114 described in the foregoing.

The processor 316 is configured to read from and write to the memory 315 and the processor 316 is configured to read from and write to the memory 315. Although the processor 316 is depicted as a single component, the processor 316 may be implemented as one or more separate components. Similarly, although the memory 315 is illustrated as a single component, the memory 315 may be implemented as one or more separate components, some or all of which may be integrated/removable and/or may provide permanent / semi-permanent/ dynamic/cached storage.

The memory 315 may store the computer program 317 comprising computer-executable instructions that control the operation of the apparatus 300 when loaded into the processor 316. As an example, the computer program 317 may include one or more sequences of one or more instructions. The computer program 317 may be provided as a computer program code. The processor 316 is able to load and execute the computer program 317 by reading the one or more sequences of one or more instructions included therein from the memory 315. The one or more sequences of one or more instructions may be configured to, when executed by the processor 316, cause the apparatus 300 to carry out operations, procedures and/or functions described in the foregoing in context of the user device 110. Hence, the apparatus 300 may comprise at least one processor 316 and at least one memory 315 including computer program code for one or more programs, the at least one memory 315 and the computer program code configured to, with the at least one processor 316, cause the apparatus 300 to perform operations, procedures and/or functions described in the foregoing in context of the user device 110.

The computer program 317 may be provided e.g. as a computer program product comprising at least one computer-readable non-transitory medium having program code stored thereon, the program code, when executed by the apparatus 300, causing the apparatus 300 at least to perform operations, procedures and/or functions described in the foregoing in context of the user device 110. The computer-readable non-transitory medium may comprise a memory device or a record medium such as a CD-ROM, a DVD, a Blu-ray disc or another article of manufacture that tangibly embodies the computer program. As another example, the computer program 317 may be provided as a signal configured to reliably transfer the computer program 317. Reference(s) to a processor should not be understood to encompass only programmable processors, but also dedicated circuits such as field-programmable gate arrays (FPGA), application specific circuits (ASIC), signal processors, etc.

Features described in the preceding description may be used in combinations other than the combinations explicitly described. Although functions have been described with reference to certain features, those functions may be performable by other features whether described or not. Although features have been described with reference to certain embodiments, those features may also be present in other embodiments whether described or not.

## Claims

1. A method for clothing selection in an electronic device, the method comprising
obtaining activity information that identifies a type of an activity a user is planning to carry out;
obtaining ambience information pertaining to a location of said activity, which ambience information characterizes ambient conditions in said location;
deriving, on basis of said ambience information in dependence of said activity information, a target value for a clothing indicator that is descriptive of a required overall insulation level provided by one or more garments for said activity;
receiving, over a wireless connection from one or more garments provided with wireless communication means, a respective value of said clothing indicator assigned for a garment selected by the user;
deriving, for said activity, an accumulated value of the clothing indictors received from said garments selected by the user; and
providing, via a user interface of said electronic device, on basis of a comparison of said accumulated value and said target value, an indication regarding a suitable clothing for said activity.

2. A method according to claim 1, wherein said providing comprises one or more of the following:
providing an indication regarding a required amount of clothing for said activity having been reached or exceeded in response to said accumulated value reaching or exceeding said target value;
providing an indication regarding a required amount of clothing for said activity having not been reached in response to said accumulated value failing to reach said target value; and
providing an indication regarding an optimal amount of clothing for said activity having been reached in response to said accumulated value being equal or substantially equal to said target value.

3. A method according to any of claims 1 to 2, wherein said derivation of the target value comprises deriving a local target value on basis of mapping information available in a local database stored in said electronic device.

4. A method according to any of claims 1 to 3, wherein said derivation of the target value comprises obtaining a reference value for said clothing indicator, comprising
transmitting a request for the reference value to a server,
receiving, from the server, the reference value derived in accordance with the request on basis of mapping information available in a remote database stored in the server, and
using the received reference value as said target value.

5. A method according to claim 4, wherein said request comprises one or more personal characteristics of the user of the electronic device and at least one further characteristic pertaining to said activity, wherein deriving the reference value in the server comprises
searching the remote database to identify database entries that indicate personal characteristics that are similar to those received in said request and that indicate further characteristics that match those received in said request, and
deriving the reference value on basis of the database entries identified in the search.

6. A method according to any of claims 1 to 5, wherein said ambience information comprises one or more of a temperature in said location and humidity in said location.

7. A method according to any of claims 1 to 6, wherein said clothing indicator comprises a clo-value that is descriptive of the amount of thermal insulation one or more garments are estimated to provide for a user wearing said one or more garments.

8. A method according to any of claims 1 to 7, wherein the value of said clothing indicator is received from a garment in response to one or more of the following
a sensor means provided in said garment detecting a state that indicates that the user has put on the garment, and
bringing the electronic device in proximity of the wireless communication means provided in said garment.

9. A method according to any of claims 1 to 8, wherein said provision of the indication comprises one or more of the following:
playing back an alarm sound via audio reproduction means of the electronic device,
displaying a visual indication in a display of the electronic device,
activating a vibratory component in the electronic device, or
causing one of the garments selected by the user to activate a vibratory component included therein.

10. A method according to any of claims 1 to 9, further comprising
measuring, during said activity by using sensor means provided in the electronic device, one or more characteristics that are indicative of the insulation level provided by one or more a number of garments worn by the user, and
transmitting, after said activity to a server, a message comprising said measured one or more characteristics, one or more personal characteristics of the user, the accumulated value of the clothing level indictors to for storage in a remote database stored in the server.

11. A method according to claim 10, wherein said message further comprises at least one of the following for storage in said remote database:
the weather information,
the activity information,
time and date of the activity, and
the location of said activity

12. A method according to any of claims 1 to 11,
wherein said obtaining activity information comprises obtaining activity information that identifies respective types of a plurality of activities the user is planning to carry out;
wherein said steps of obtaining the ambience information, deriving the target value, receiving the value for the clothing indicator, deriving the accumulated value and providing the indication are carried out separately for each of said plurality of activities.

13. A computer program product, comprising computer readable program code tangibly embodied on a non-transitory computer readable medium, the program code configured to cause performing the method according to any of claims 1 to 12 when run on a computing apparatus.

14. An apparatus, comprising means for performing the method of at least one of claims 1 to 12.
